# EUROPEAN PATENT APPLICATION

(11) **EP 2 157 459 A1**
(43) Date of publication of application: **24.02.2010**
(21) Application number: 07788628.1
(22) Date of filing: 26.06.2007
(51) Int. Cl.: G02B 5/23, A61F 9/00, E04B 1/62

(54) **PREVENTIVE ELEMENT INTENDED TO BE APPLIED TO TRANSPARENT BUILDING SURFACES IN ORDER TO PROVIDE EYE PROTECTION AND THERAPY**

(30) Priority: 01.06.2007 ES 200701515
(71) Applicant: Universidad Complutense De Madrid, 28040 Madrid (ES)
(72) Inventor: SANCHEZ RAMOS, Celia, E-28040 Madrid (ES)
(74) Representative: Temino Ceniceros, Ignacio
(86) International application number: PCT/ES2007/000380
(87) International publication number: WO 2008/145769

(57) **Abstract**

Element for the protection and therapy of eyes applied to the transparent vertical surfaces of buildings. The invention is prepared by applying a yellow pigmented filter to the transparent or translucent surfaces of a building in which a subject may be found, and is designed to protect the eyes from the short wavelengths of the visible spectrum in the range 500 to 380 nm. The invention avoids the difficulties and risks of existing ways of protecting healthy eyes or eyes subjected to cataract surgery, and improves the protection of eyes suffering neurodegeneration, simply by applying a filter to the transparent or translucent surfaces of any building in which a subject may be found to provide protection against the neurodegenerative components of light (short wavelengths).

## Description

The invention is intended for the ophthalmology sector of the market, within the area of optical applications of a therapeutic and preventive nature. The invention generally provides a transparent filtering optic medium, or yellow pigment, applied to the transparent or translucent vertical surfaces of buildings to protect healthy eyes and/or pseudoaphakic eyes (eyes that have undergone cataract surgery) and/or eyes with retinal degeneration from the short wavelengths of the visible spectrum from 500 to 380 nm that cause retinal degeneration. As an example, this filtering component is indicated for use in windows for houses, offices, and shops.

### BACKGROUND OF THE INVENTION

Visual perception is the result of the response to visible radiation in the wavelength range 380-760 nm. In the environment, solar radiation is the main risk factor for vision. The sun emits UV rays and IR radiation, which are mainly absorbed by the atmosphere. When the solar radiation transmitted through the atmosphere reaches the Earth's surface it consists of UV-B rays (230-300 nm), UV or UV-A rays (300-380 nm), visible light rays (380-760 nm) and IR rays (760-1400 nm). Healthy human eyes freely transmit IR rays and those of most of the visible spectrum to the retina, but the cornea and crystalline lens prevent the most reactive wavelengths of the visible spectrum (UV-B rays and the blue portion of the spectrum) from reaching the retina.

The human crystalline lens changes its transmission properties as it ages by intensifying its yellowish color thus increasing its capacity to filter out UV and blue light rays. Hence, in persons older than 65 years, violet light (<400 nm) is not transmitted and the transmission of blue light (400-500 nm) is markedly reduced.

The retina is capable of protecting itself from short wavelengths of light in two ways: through its uneven distribution of photoreceptors, such that there are no photoreceptors sensitive to blue light in the macular depression; and through the actions of yellow pigments in this zone, which also exert a protective effect.

These natural protection systems the human eye has against the shorter wavelengths of light--the crystalline lens and structures of the retina--can be seriously affected by certain diseases and/or surgical procedures:
- Cataracts, whose surgical treatment involves the removal of the crystalline lens.
- Additionally, it is common to find a pathological ageing process that causes degradation of the retinal structures producing age-related macular degeneration (AMD).

We should also consider that both cataracts and AMD can coexist in persons older than 65 years. In this population of elderly subjects, cataract is the main cause of vision loss and AMD is the main cause of blindness. In addition we should expect an increase in both these diseases due, among other factors, to our increased life expectancy. This translates into a great interest in these diseases and their treatment options in the research field and optics industry.

Several epidemiological studies have evaluated the relationship between cataract surgery and AMD. Thus, Klein (Klein R, Klein B E, Wong T Y, Tomany S C, Cruickshanks K J. The association of cataract and cataract surgery with the long-term incidence of age-related maculopathy. Arch Ophthalmol 120:1551-1558.2002) and Freeman (Freeman E, Munoz B, West S K, Tielsch J M, Schein O D. Is there an association between cataract surgery and age-related macular degeneration? Am J Ophthalmolm 135(6): 849-856.2003) claim there is a higher risk of developing symptoms of AMD in persons who have undergone cataract surgery. However, in earlier investigations by Wang (Wang J J, Mitchell P, Cumming R G, Lim R. Cataract and age-related maculopathy: the Blue Mountains Eye Study. Ophthalmic Epidemiol 6: 317-326.1999) and McCarty (McCarty C A, Mukesh B N, Fu C L, Mitchell P, Wang J J, Taylor H R. Risks factors for age-related maculopathy: the Visual Impairment Project. Arch Ophthalmol 119:1455-1462.2001) this hypothesis was rejected, possibly because of the less developed technology used for their diagnostic measurements. Techniques such as optical coherence tomography that allow the accurate, rapid and non-invasive follow up of retinal neurodegeneration processes have only recently been introduced. These techniques are essential for establishing the determining effect of the natural pigments that absorb harmful radiations.

Several techniques have also been developed to protect eyes subjected to cataract surgery from short wavelengths of light:
- There are several types of filter containing a yellow pigment on the market yet there is no optimal procedure and/or device to apply these filters to the human eye as a preventive and/for therapeutic measure to replace and/or improve the eye's natural protection.
- Since the mid-1990s, eyes undergoing cataract extraction have been implanted with intraocular lenses containing a yellow pigment to act as a filter. This option requires surgical intervention with all its risks and difficulties. There is also a large population of subjects who have been implanted with a transparent lens to replace the natural lens during cataract surgery who are therefore devoid of the necessary protection. In these patients, the artificial crystalline lens, lacking a yellow pigment, needs to be complemented with a system to support the yellow pigment, for example, an ophthalmologic lens or contact lens.

Several patents related to the state of this technique have been developed (for healthy, pseudoaphakic eyes and/or eyes undergoing neurodegeneration) although they differ considerably from the object of the present invention:
- Methods and optical media designed to improve or modify color vision and the procedure used to prepare them (U.S. Pat. No. 5,774,202), based on the use of a color filter of a specific transmission range on any surface including glass.
- Electronic system (patent DE10023765) to be used as a window, separation or protection screen, light filter or other that allows electro-conduction.
- Window that transmits electromagnetic radiation (patent JP8210042).
- Window that transmits infra-red radiation for heat pressure environments (U.S. Pat. No. 3,633,984).
- Photochromic window that filters a portion of actinic radiation (patent GB1111977).
- Visual distinction filter (patent JP61087106) to prevent the change in luminosity that follows a change in tone and reduce demands on the eyes providing the maximum absorption possible.
- Ultraviolet filter and glass composed of this filter (patent JP10020347), whose transmittance of UV radiation is variable and adaptable.
- Electromagnetic filter (patents JP2000349542 and JP2000349541) that protects against certain magnetic frequencies received, for example, through windows.
- Absorption filter for color exposure systems (U.S. Pat. No. 5,121,030) that through the use of dyes improves visibility in conditions of intense luminosity.
- Filter that improves colors and method of use to improve human vision (U.S. Pat. No. 6,158,865). The device includes a filter that improves vision in all light environments, even extreme ambient light and low illumination levels, and an adapting ring for the filter.
- Special optical filters for certain activities and optical accessories that use these filters (U.S. Pat. No. 6,893,127) to improve the visualization of objects, for example, in sports activities.

These devices differ from the present invention mainly in their purpose and utility since none has been designed to protect eyes from short wavelengths of the visible spectrum to prevent and reduce neurodegenerative retinal processes.

### DESCRIPTION OF THE INVENTION

The aim of the invention is generally to protect and prevent eyes from absorbing blue and violet light by means of a filter applied to the transparent or translucent surfaces of a building. As mentioned above, it is particularly useful in the case of pseudophakic persons, to functionally compensate for their lack of protective pigments (removed during surgery), and as prophylaxis for subjects suffering neurodegeneration. Both these conditions are common among elderly persons but the invention is equally important for protecting healthy eyes in any subject.

The invention is prepared by applying to the transparent or translucent surfaces of a building, in which the subject may be found, a yellow filter that absorbs short wavelengths from 500 to 380 nm. As an example, this filter could be applied to the windows of houses, offices or shops.

The invention thus combines three components:
- The transparent or translucent surfaces of any building (home, office, shop or other).
- A mounting frame or system to apply the filter to the transparent surface.
- A yellow dye of those available on the market that is compatible with the surface material and absorbs short wavelengths, from 500 to 380 nm, across the entire light transmitting area of the surface.

### DETAILED DESCRIPTION OF THE INVENTION AND EXAMPLE

There are several ways of manufacturing the invention depending on the material of the surface to which the filter will be applied. The instructions below are provided as an example for use in the glass of a house window, but are in no way restrictive.

The following layers of glass can be used to manufacture the window: transparent monolithic glass of 90% Tvis (visible light transmittance); yellow-tinted monolithic glass of 62% Tvis, laminated monolithic glass of 88% Tvis, transparent satin glass of 82% Tvis, yellow-tinted satin glass of 56% Tvis, laminated satin glass of 80% Tvis.

Thus, by combining several transparent or translucent surfaces of a building in which a subject may be found with a yellow filter, any individual can protect his/her healthy eyes from short wavelengths of light and patients who have undergone cataract extraction and the implant of a transparent intraocular lens can correct their lack of natural protection. Moreover, eyes with neurodegeneration can improve since the invention enhances their natural protection. In this way, the problems related to the technical options available on the market can be avoided (filters with no application system and intraocular lenses).

## Claims

1. Element for the protection of healthy eyes from short wavelengths of light **characterised by** being the result of applying a yellow pigment, which absorbs short wavelengths from 500 to 380 nm, to the transparent or translucent surfaces of one or several buildings.

2. Element for the protection of healthy eyes from short wavelengths of light according to claim 1 that comprises a yellow pigment dye appropriate for its application to transparent or translucent surfaces.

3. Element for the protection of healthy eyes from short wavelengths of light according to claim 1 that comprises one or several transparent or translucent surfaces of a building.

4. Element for the protection of healthy eyes from short wavelengths of light according to claim 1 in which the transparent or translucent surfaces are the windows of houses, offices, shops or other buildings.

5. Element according to claim 1 **characterised by** being filtering and transparent.

6. Therapeutic and prophylactic element for pseudoaphakic eyes **characterised by** being the result of applying a yellow pigment filter, which absorbs short wave lengths from 500 to 380 nm to the transparent or translucent surfaces on one or several buildings.

7. Therapeutic and prophylactic element for pseudoaphakic eyes according to claim 6 that comprises a filter with a yellow pigment appropriate for use on transparent or translucent surfaces.

8. Therapeutic and prophylactic element for pseudoaphakic eyes according to claim 6 that comprises one or several transparent or translucent surfaces of a building.

9. Therapeutic and prophylactic element for pseudoaphakic eyes according to claim 6 in which the transparent or translucent surfaces are the windows of houses, offices, shops or other buildings.

10. Element according to claim 6 **characterised by** being filtering and transparent.

11. Therapeutic and prophylactic element for eyes with retinal neurodegeneration **characterised by** being the result of applying a yellow pigment filter, which absorbs short wave lengths from 500 to 380 nm, to the transparent or translucent surfaces on one or several buildings.

12. Therapeutic and prophylactic element for eyes with retinal neurodegeneration according to claim 11 that comprises a yellow pigment dye appropriate for use on transparent or translucent surfaces.

13. Therapeutic and prophylactic element for eyes with retinal neurodegeneration according to claim 11 that comprises one or several transparent or translucent surfaces of a building.

14. Therapeutic and prophylactic element for eyes with retinal neurodegeneration according to claim 11 in which the transparent or translucent surfaces are the windows of homes, offices, shops or other buildings.

15. Element according to claim 11 **characterised by** being filtering and transparent.
